# EUROPEAN PATENT APPLICATION

(11) **EP 3 904 374 A1**
(43) Date of publication of application: **03.11.2021**
(21) Application number: 19904404.1
(22) Date of filing: 26.12.2019
(51) Int. Cl.: C07K 14/705, C12N 5/10, C12N 15/12, C12N 15/63

(54) **T-CELL RECEPTOR MODIFIED OBJECT**

(30) Priority: 27.12.2018 JP 2018245253
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); KASSAI, Yoshiaki, Fujisawa-shi, Kanagawa 251-0012 (JP); HAYASHI, Akira, Fujisawa-shi, Kanagawa 251-0012 (JP)
(74) Representative: Hoffmann Eitle
(86) International application number: PCT/JP2019/051057
(87) International publication number: WO 2020/138256

(57) **Abstract**

The present invention provides a variant of a T-cell receptor containing a combination of two polypeptides containing a constant region of a T cell receptor chain selected from the group consisting of α chain, β chain, γ chain and δ chain, wherein the polypeptide does not contain a complementarity determining region (CDR) of the T cell receptor chain, a complementarity determining region (CDR) of the α chain, and a complementarity determining region (CDR) of the β chain.

SEQUENCE LISTING

<110>  Kyoto University
       Takeda Pharmaceutical Company Limited

<120>  Variant of T-cell receptor

<130>  092982

<150>  JP 2018-245253
<151>  2018-12-27

<160>  15

<170>  PatentIn version 3.5

<210>  1
<211>  141
<212>  PRT
<213>  Artificial Sequence

<220>
<223>  constant region of TCR alpha chain

<400>  1

Val Ile Gln Asn Pro Asp Pro Ala Val Tyr Gln Leu Arg Asp Ser Lys
1               5                   10                  15

Ser Ser Asp Lys Ser Val Cys Leu Phe Thr Asp Phe Asp Ser Gln Thr
            20                  25                  30

Asn Val Ser Gln Ser Lys Asp Ser Asp Val Tyr Ile Thr Asp Lys Thr
        35                  40                  45

Val Leu Asp Met Arg Ser Met Asp Phe Lys Ser Asn Ser Ala Val Ala
    50                  55                  60

Trp Ser Asn Lys Ser Asp Phe Ala Cys Ala Asn Ala Phe Asn Asn Ser
65                  70                  75                  80

Ile Ile Pro Glu Asp Thr Phe Phe Pro Ser Pro Glu Ser Ser Cys Asp
                85                  90                  95

Val Lys Leu Val Glu Lys Ser Phe Glu Thr Asp Thr Asn Leu Asn Phe
            100                 105                 110

Gln Asn Leu Ser Val Ile Gly Phe Arg Ile Leu Leu Leu Lys Val Ala
        115                 120                 125

Gly Phe Asn Leu Leu Met Thr Leu Arg Leu Trp Ser Ser
    130                 135                 140

<210>  2

<211>  175
<212>  PRT
<213>  Artificial Sequence

<220>
<223>  constant region of TCR beta chain

<400>  2

Asp Leu Asn Lys Val Phe Pro Pro Glu Val Ala Val Phe Glu Pro Ser
1               5                   10                  15

Glu Ala Glu Ile Ser His Thr Gln Lys Ala Thr Leu Val Cys Leu Ala
            20                  25                  30

Thr Gly Phe Phe Pro Asp His Val Glu Leu Ser Trp Trp Val Asn Gly
        35                  40                  45

Lys Glu Val His Ser Gly Val Ser Thr Asp Pro Gln Pro Leu Lys Glu
    50                  55                  60

Gln Pro Ala Leu Asn Asp Ser Arg Tyr Cys Leu Ser Ser Arg Leu Arg
65                  70                  75                  80

Val Ser Ala Thr Phe Trp Gln Asn Pro Arg Asn His Phe Arg Cys Gln
                85                  90                  95

Val Gln Phe Tyr Gly Leu Ser Glu Asn Asp Glu Trp Thr Gln Asp Arg
            100                 105                 110

Ala Lys Pro Val Thr Gln Ile Val Ser Ala Glu Ala Trp Gly Arg Ala
        115                 120                 125

Asp Cys Gly Phe Thr Ser Val Ser Tyr Gln Gln Gly Val Leu Ser Ala
    130                 135                 140

Thr Ile Leu Tyr Glu Ile Leu Leu Gly Lys Ala Thr Leu Tyr Ala Val
145                 150                 155                 160

Leu Val Ser Ala Leu Val Leu Met Ala Met Val Lys Arg Lys Asp
                165                 170                 175


<210>  3
<211>  177
<212>  PRT
<213>  Artificial Sequence

<220>
<223>  constant region of TCR beta chain

<400>  3

Leu Lys Asn Val Phe Pro Pro Glu Val Ala Val Phe Glu Pro Ser Glu
1               5                   10                  15

Ala Glu Ile Ser His Thr Gln Lys Ala Thr Leu Val Cys Leu Ala Thr
            20                  25                  30

Gly Phe Tyr Pro Asp His Val Glu Leu Ser Trp Trp Val Asn Gly Lys
        35                  40                  45

Glu Val His Ser Gly Val Ser Thr Asp Pro Gln Pro Leu Lys Glu Gln
    50                  55                  60

Pro Ala Leu Asn Asp Ser Arg Tyr Cys Leu Ser Ser Arg Leu Arg Val
65                  70                  75                  80

Ser Ala Thr Phe Trp Gln Asn Pro Arg Asn His Phe Arg Cys Gln Val
                85                  90                  95

Gln Phe Tyr Gly Leu Ser Glu Asn Asp Glu Trp Thr Gln Asp Arg Ala
            100                 105                 110

Lys Pro Val Thr Gln Ile Val Ser Ala Glu Ala Trp Gly Arg Ala Asp
        115                 120                 125

Cys Gly Phe Thr Ser Glu Ser Tyr Gln Gln Gly Val Leu Ser Ala Thr
    130                 135                 140

Ile Leu Tyr Glu Ile Leu Leu Gly Lys Ala Thr Leu Tyr Ala Val Leu
145                 150                 155                 160

Val Ser Ala Leu Val Leu Met Ala Met Val Lys Arg Lys Asp Ser Arg
                165                 170                 175

Gly

<210>  4
<211>  21
<212>  PRT
<213>  Artificial Sequence

<220>
<223>  CD8 signal sequence

<400>  4

Met Ala Leu Pro Val Thr Ala Leu Leu Leu Pro Leu Ala Leu Leu Leu
1               5                   10                  15

His Ala Ala Arg Pro

            20

<210>  5
<211>  19
<212>  PRT
<213>  Artificial Sequence

<220>
<223>  IGH signal sequence

<400>  5

Met Asp Trp Thr Trp Arg Ile Leu Phe Leu Val Ala Ala Ala Thr Gly
1               5                   10                  15

Ala His Ser

<210>  6
<211>  1143
<212>  DNA
<213>  Artificial Sequence

<220>
<223>  base sequence of AB5

<400>  6
atggcccttc cggtaacagc tctcctcctt cctttggctc tcctgctcca tgccgcccgg      60

cccgaggacc tgaacaaggt gttcccaccc gaggtcgctg tgtttgagcc atcagaagca     120

gagatctccc acacccaaaa ggccacactg gtgtgcctgg ccacaggctt cttccctgac     180

cacgtggagc tgagctggtg ggtgaatggg aaggaggtgc acagtggggt cagcacggac     240

ccgcagcccc tcaaggagca gcccgccctc aatgactcca gatactgcct gagcagccgc     300

ctgagggtct cggccacctt ctggcagaac ccccgcaacc acttccgctg tcaagtccag     360

ttctacgggc tctcggagaa tgacgagtgg acccaggata gggccaaacc cgtcacccag     420

atcgtcagcg ccgaggcctg gggtagagca gactgtggct ttacctcggt gtcctaccag     480

caaggggtcc tgtctgccac catcctctat gagatcctgc tagggaaggc caccctgtat     540

gctgtgctgg tcagcgccct tgtgttgatg gccatggtca agagaaagga ttccggaagc     600

ggagccacca acttcagcct gctgaagcag gccggtgacg tcgaggagaa tcctggcccc     660

atggactgga catggaggat actgttcctg gtagccgcag ctactggcgc ccattccgta     720

atccagaacc ctgaccctgc cgtgtaccag ctgagagact ctaaatccag tgacaagtct     780

gtctgcctat tcaccgattt tgattctcaa acaaatgtgt cacaaagtaa ggattctgat     840

gtgtatatca cagacaaaac tgtgctagac atgaggtcta tggacttcaa gagcaacagt     900

gctgtggcct ggagcaacaa atctgacttt gcatgtgcaa acgccttcaa caacagcatt     960

attccagaag acaccttctt ccccagccca gaaagttcct gtgatgtcaa gctggtcgag    1020

aaaagctttg aaacagatac gaacctaaac tttcaaaacc tgtcagtgat tgggttccga     1080

atcctcctcc tgaaagtggc cgggtttaat ctgctcatga cgctgcggct gtggtccagc     1140

tga                                                                   1143

<210>  7
<211>  197
<212>  PRT
<213>  Artificial Sequence

<220>
<223>  amino acid sequence of CD8 and TRBC1 of AB5

<400>  7

Met Ala Leu Pro Val Thr Ala Leu Leu Leu Pro Leu Ala Leu Leu Leu
1               5                   10                  15

His Ala Ala Arg Pro Glu Asp Leu Asn Lys Val Phe Pro Pro Glu Val
            20                  25                  30

Ala Val Phe Glu Pro Ser Glu Ala Glu Ile Ser His Thr Gln Lys Ala
        35                  40                  45

Thr Leu Val Cys Leu Ala Thr Gly Phe Phe Pro Asp His Val Glu Leu
    50                  55                  60

Ser Trp Trp Val Asn Gly Lys Glu Val His Ser Gly Val Ser Thr Asp
65                  70                  75                  80

Pro Gln Pro Leu Lys Glu Gln Pro Ala Leu Asn Asp Ser Arg Tyr Cys
                85                  90                  95

Leu Ser Ser Arg Leu Arg Val Ser Ala Thr Phe Trp Gln Asn Pro Arg
            100                 105                 110

Asn His Phe Arg Cys Gln Val Gln Phe Tyr Gly Leu Ser Glu Asn Asp
        115                 120                 125

Glu Trp Thr Gln Asp Arg Ala Lys Pro Val Thr Gln Ile Val Ser Ala
    130                 135                 140

Glu Ala Trp Gly Arg Ala Asp Cys Gly Phe Thr Ser Val Ser Tyr Gln
145                 150                 155                 160

Gln Gly Val Leu Ser Ala Thr Ile Leu Tyr Glu Ile Leu Leu Gly Lys
                165                 170                 175

Ala Thr Leu Tyr Ala Val Leu Val Ser Ala Leu Val Leu Met Ala Met

180                 185                 190

Val Lys Arg Lys Asp
        195

<210>  8
<211>  160
<212>  PRT
<213>  Artificial Sequence

<220>
<223>  amino acid sequence of IGH and TRAC of AB5 or AB7

<400>  8

Met Asp Trp Thr Trp Arg Ile Leu Phe Leu Val Ala Ala Ala Thr Gly
1               5                   10                  15

Ala His Ser Val Ile Gln Asn Pro Asp Pro Ala Val Tyr Gln Leu Arg
            20                  25                  30

Asp Ser Lys Ser Ser Asp Lys Ser Val Cys Leu Phe Thr Asp Phe Asp
        35                  40                  45

Ser Gln Thr Asn Val Ser Gln Ser Lys Asp Ser Asp Val Tyr Ile Thr
    50                  55                  60

Asp Lys Thr Val Leu Asp Met Arg Ser Met Asp Phe Lys Ser Asn Ser
65                  70                  75                  80

Ala Val Ala Trp Ser Asn Lys Ser Asp Phe Ala Cys Ala Asn Ala Phe
                85                  90                  95

Asn Asn Ser Ile Ile Pro Glu Asp Thr Phe Phe Pro Ser Pro Glu Ser
            100                 105                 110

Ser Cys Asp Val Lys Leu Val Glu Lys Ser Phe Glu Thr Asp Thr Asn
        115                 120                 125

Leu Asn Phe Gln Asn Leu Ser Val Ile Gly Phe Arg Ile Leu Leu Leu
    130                 135                 140

Lys Val Ala Gly Phe Asn Leu Leu Met Thr Leu Arg Leu Trp Ser Ser
145                 150                 155                 160

<210>  9
<211>  1143
<212>  DNA
<213>  Artificial Sequence

<220>

<223>  base sequence of AB6

<400>  9
atggcccttc cggtaacagc tctcctcctt cctttggctc tcctgctcca tgccgcccgg      60

cccgaggacc tgaacaaggt gttcccaccc gaggtcgctg tgtttgagcc atcagaagca     120

gagatctccc acacccaaaa ggccacactg gtgtgcctgg ccacaggctt cttccctgac     180

cacgtggagc tgagctggtg ggtgaatggg aaggaggtgc acagtggggt ctgcacggac     240

ccgcagcccc tcaaggagca gcccgccctc aatgactcca gatactgcct gagcagccgc     300

ctgagggtct cggccacctt ctggcagaac ccccgcaacc acttccgctg tcaagtccag     360

ttctacgggc tctcggagaa tgacgagtgg acccaggata gggccaaacc cgtcacccag     420

atcgtcagcg ccgaggcctg gggtagagca gactgtggct ttacctcggt gtcctaccag     480

caaggggtcc tgtctgccac catcctctat gagatcctgc tagggaaggc caccctgtat     540

gctgtgctgg tcagcgccct tgtgttgatg gccatggtca agagaaagga ttccggaagc     600

ggagccacca acttcagcct gctgaagcag gccggtgacg tcgaggagaa tcctggcccc     660

atggactgga catggaggat actgttcctg gtagccgcag ctactggcgc ccattccgta     720

atccagaacc ctgaccctgc cgtgtaccag ctgagagact ctaaatccag tgacaagtct     780

gtctgcctat tcaccgattt tgattctcaa acaaatgtgt cacaaagtaa ggattctgat     840

gtgtatatca cagacaaatg tgtgctagac atgaggtcta tggacttcaa gagcaacagt     900

gctgtggcct ggagcaacaa atctgacttt gcatgtgcaa acgccttcaa caacagcatt     960

attccagaag acaccttctt ccccagccca gaaagttcct gtgatgtcaa gctggtcgag    1020

aaaagctttg aaacagatac gaacctaaac tttcaaaacc tgtcagtgat tgggttccga    1080

atcctcctcc tgaaagtggc cgggtttaat ctgctcatga cgctgcggct gtggtccagc    1140

tga                                                                  1143


<210>  10
<211>  197
<212>  PRT
<213>  Artificial Sequence

<220>
<223>  amino acid sequence of CD8 and TRBC1 of AB6

<400>  10

Met Ala Leu Pro Val Thr Ala Leu Leu Leu Pro Leu Ala Leu Leu Leu
1               5                   10                  15


His Ala Ala Arg Pro Glu Asp Leu Asn Lys Val Phe Pro Pro Glu Val
            20                  25                  30


Ala Val Phe Glu Pro Ser Glu Ala Glu Ile Ser His Thr Gln Lys Ala

35                  40                  45

Thr Leu Val Cys Leu Ala Thr Gly Phe Phe Pro Asp His Val Glu Leu
    50                  55                  60

Ser Trp Trp Val Asn Gly Lys Glu Val His Ser Gly Val Cys Thr Asp
65                  70                  75                  80

Pro Gln Pro Leu Lys Glu Gln Pro Ala Leu Asn Asp Ser Arg Tyr Cys
                85                  90                  95

Leu Ser Ser Arg Leu Arg Val Ser Ala Thr Phe Trp Gln Asn Pro Arg
            100                 105                 110

Asn His Phe Arg Cys Gln Val Gln Phe Tyr Gly Leu Ser Glu Asn Asp
        115                 120                 125

Glu Trp Thr Gln Asp Arg Ala Lys Pro Val Thr Gln Ile Val Ser Ala
    130                 135                 140

Glu Ala Trp Gly Arg Ala Asp Cys Gly Phe Thr Ser Val Ser Tyr Gln
145                 150                 155                 160

Gln Gly Val Leu Ser Ala Thr Ile Leu Tyr Glu Ile Leu Leu Gly Lys
                165                 170                 175

Ala Thr Leu Tyr Ala Val Leu Val Ser Ala Leu Val Leu Met Ala Met
            180                 185                 190

Val Lys Arg Lys Asp
        195

<210>  11
<211>  160
<212>  PRT
<213>  Artificial Sequence

<220>
<223>  amino acid sequence of IGH and TRAC of AB6 or AB8

<400>  11

Met Asp Trp Thr Trp Arg Ile Leu Phe Leu Val Ala Ala Ala Thr Gly
1               5                   10                  15

Ala His Ser Val Ile Gln Asn Pro Asp Pro Ala Val Tyr Gln Leu Arg
            20                  25                  30

Asp Ser Lys Ser Ser Asp Lys Ser Val Cys Leu Phe Thr Asp Phe Asp
        35                  40                  45

Ser Gln Thr Asn Val Ser Gln Ser Lys Asp Ser Asp Val Tyr Ile Thr
    50                  55                  60

Asp Lys Cys Val Leu Asp Met Arg Ser Met Asp Phe Lys Ser Asn Ser
65                  70                  75                  80

Ala Val Ala Trp Ser Asn Lys Ser Asp Phe Ala Cys Ala Asn Ala Phe
                85                  90                  95

Asn Asn Ser Ile Ile Pro Glu Asp Thr Phe Phe Pro Ser Pro Glu Ser
            100                 105                 110

Ser Cys Asp Val Lys Leu Val Glu Lys Ser Phe Glu Thr Asp Thr Asn
        115                 120                 125

Leu Asn Phe Gln Asn Leu Ser Val Ile Gly Phe Arg Ile Leu Leu Leu
    130                 135                 140

Lys Val Ala Gly Phe Asn Leu Leu Met Thr Leu Arg Leu Trp Ser Ser
145                 150                 155                 160

<210>  12
<211>  1149
<212>  DNA
<213>  Artificial Sequence

<220>
<223>  base sequence of AB7

<400>  12
atggcccttc cggtaacagc tctcctcctt cctttggctc tcctgctcca tgccgcccgg      60

cccgacctga aaaacgtgtt cccacccgag gtcgctgtgt ttgagccatc agaagcagag     120

atctcccaca cccaaaaggc cacactggtg tgcctggcca caggcttcta ccccgaccac     180

gtggagctga gctggtgggt gaatgggaag gaggtgcaca gtggggtcag cacagacccg     240

cagcccctca aggagcagcc cgccctcaat gactccagat actgcctgag cagccgcctg     300

agggtctcgg ccaccttctg gcagaacccc cgcaaccact tccgctgtca agtccagttc     360

tacgggctct cggagaatga cgagtggacc caggataggg ccaaacctgt cacccagatc     420

gtcagcgccg aggcctgggg tagagcagac tgtggcttca cctccgagtc ttaccagcaa     480

ggggtcctgt ctgccaccat cctctatgag atcttgctag ggaaggccac cttgtatgcc     540

gtgctggtca gtgccctcgt gctgatggcc atggtcaaga gaaaggattc cagaggctcc     600

ggaagcggag ccaccaactt cagcctgctg aagcaggccg gtgacgtcga ggagaatcct     660

ggccccatgg actggacatg gaggatactg ttcctggtag ccgcagctac tggcgcccat     720

tccgtaatcc agaaccctga ccctgccgtg taccagctga gagactctaa atccagtgac 780

aagtctgtct gcctattcac cgattttgat tctcaaacaa atgtgtcaca aagtaaggat 840

tctgatgtgt atatcacaga caaaactgtg ctagacatga ggtctatgga cttcaagagc 900

aacagtgctg tggcctggag caacaaatct gactttgcat gtgcaaacgc cttcaacaac 960

agcattattc cagaagacac cttcttcccc agcccagaaa gttcctgtga tgtcaagctg 1020

gtcgagaaaa gctttgaaac agatacgaac ctaaactttc aaaacctgtc agtgattggg 1080

ttccgaatcc tcctcctgaa agtggccggg tttaatctgc tcatgacgct gcggctgtgg 1140

tccagctga 1149

<210> 13
<211> 199
<212> PRT
<213> Artificial Sequence

<220>
<223> amino acid sequence of CD8 and TRBC2 of AB7

<400> 13

Met Ala Leu Pro Val Thr Ala Leu Leu Leu Pro Leu Ala Leu Leu Leu
1               5                   10                  15

His Ala Ala Arg Pro Asp Leu Lys Asn Val Phe Pro Pro Glu Val Ala
            20                  25                  30

Val Phe Glu Pro Ser Glu Ala Glu Ile Ser His Thr Gln Lys Ala Thr
        35                  40                  45

Leu Val Cys Leu Ala Thr Gly Phe Tyr Pro Asp His Val Glu Leu Ser
    50                  55                  60

Trp Trp Val Asn Gly Lys Glu Val His Ser Gly Val Ser Thr Asp Pro
65                  70                  75                  80

Gln Pro Leu Lys Glu Gln Pro Ala Leu Asn Asp Ser Arg Tyr Cys Leu
                85                  90                  95

Ser Ser Arg Leu Arg Val Ser Ala Thr Phe Trp Gln Asn Pro Arg Asn
            100                 105                 110

His Phe Arg Cys Gln Val Gln Phe Tyr Gly Leu Ser Glu Asn Asp Glu
        115                 120                 125

Trp Thr Gln Asp Arg Ala Lys Pro Val Thr Gln Ile Val Ser Ala Glu
    130                 135                 140

Ala Trp Gly Arg Ala Asp Cys Gly Phe Thr Ser Glu Ser Tyr Gln Gln
145                 150                 155                 160

Gly Val Leu Ser Ala Thr Ile Leu Tyr Glu Ile Leu Leu Gly Lys Ala
                165                 170                 175

Thr Leu Tyr Ala Val Leu Val Ser Ala Leu Val Leu Met Ala Met Val
            180                 185                 190

Lys Arg Lys Asp Ser Arg Gly
        195

<210>  14
<211>  1149
<212>  DNA
<213>  Artificial Sequence

<220>
<223>  base sequence of AB8

<400>  14
atggcccttc cggtaacagc tctcctcctt cctttggctc tcctgctcca tgccgcccgg      60

cccgacctga aaaacgtgtt cccacccgag gtcgctgtgt ttgagccatc agaagcagag     120

atctcccaca cccaaaaggc cacactggtg tgcctggcca caggcttcta ccccgaccac     180

gtggagctga gctggtgggt gaatgggaag gaggtgcaca gtggggtctg cacagacccg     240

cagcccctca aggagcagcc cgccctcaat gactccagat actgcctgag cagccgcctg     300

agggtctcgg ccaccttctg gcagaacccc cgcaaccact tccgctgtca agtccagttc     360

tacgggctct cggagaatga cgagtggacc caggataggg ccaaacctgt cacccagatc     420

gtcagcgccg aggcctgggg tagagcagac tgtggcttca cctccgagtc ttaccagcaa     480

ggggtcctgt ctgccaccat cctctatgag atcttgctag ggaaggccac cttgtatgcc     540

gtgctggtca gtgccctcgt gctgatggcc atggtcaaga gaaaggattc cagaggctcc     600

ggaagcggag ccaccaactt cagcctgctg aagcaggccg gtgacgtcga ggagaatcct     660

ggccccatgg actggacatg gaggatactg ttcctggtag ccgcagctac tggcgcccat     720

tccgtaatcc agaaccctga ccctgccgtg taccagctga gagactctaa atccagtgac     780

aagtctgtct gcctattcac cgattttgat tctcaaacaa atgtgtcaca aagtaaggat     840

tctgatgtgt atatcacaga caaatgtgtg ctagacatga ggtctatgga cttcaagagc     900

aacagtgctg tggcctggag caacaaatct gactttgcat gtgcaaacgc cttcaacaac     960

agcattattc cagaagacac cttcttcccc agcccagaaa gttcctgtga tgtcaagctg    1020

gtcgagaaaa gctttgaaac agatacgaac ctaaactttc aaaacctgtc agtgattggg    1080

ttccgaatcc tcctcctgaa agtggccggg tttaatctgc tcatgacgct gcggctgtgg    1140

tccagctga                                                              1149

<210>  15
<211>  199
<212>  PRT
<213>  Artificial Sequence

<220>
<223>  amino acid sequence of CD8 and TRBC2 of AB8

<400>  15

Met Ala Leu Pro Val Thr Ala Leu Leu Leu Pro Leu Ala Leu Leu Leu
1               5                   10                  15

His Ala Ala Arg Pro Asp Leu Lys Asn Val Phe Pro Pro Glu Val Ala
            20                  25                  30

Val Phe Glu Pro Ser Glu Ala Glu Ile Ser His Thr Gln Lys Ala Thr
        35                  40                  45

Leu Val Cys Leu Ala Thr Gly Phe Tyr Pro Asp His Val Glu Leu Ser
    50                  55                  60

Trp Trp Val Asn Gly Lys Glu Val His Ser Gly Val Cys Thr Asp Pro
65                  70                  75                  80

Gln Pro Leu Lys Glu Gln Pro Ala Leu Asn Asp Ser Arg Tyr Cys Leu
                85                  90                  95

Ser Ser Arg Leu Arg Val Ser Ala Thr Phe Trp Gln Asn Pro Arg Asn
            100                 105                 110

His Phe Arg Cys Gln Val Gln Phe Tyr Gly Leu Ser Glu Asn Asp Glu
        115                 120                 125

Trp Thr Gln Asp Arg Ala Lys Pro Val Thr Gln Ile Val Ser Ala Glu
    130                 135                 140

Ala Trp Gly Arg Ala Asp Cys Gly Phe Thr Ser Glu Ser Tyr Gln Gln
145                 150                 155                 160

Gly Val Leu Ser Ala Thr Ile Leu Tyr Glu Ile Leu Leu Gly Lys Ala
                165                 170                 175

Thr Leu Tyr Ala Val Leu Val Ser Ala Leu Val Leu Met Ala Met Val
            180                 185                 190

Lys Arg Lys Asp Ser Arg Gly
        195

## Description

### [Technical Field]

The present invention relates to a variant of T-cell receptor, a cell expressing the variant, and the like.

### (Background of the Invention)

T cells play a central role in immune systems against foreign pathogens such as bacteria, viruses and the like and abnormal cells such as cancer cells and the like. Particularly, cytotoxic T lymphocyte (CTL) recognizes, via the T cell receptor (TCR) present on its cell surface, antigenic peptides derived from viruses or tumors, etc. which are presented together with class 1 major histocompatible antigen of antigen-presenting cells, and specifically exerts cytotoxic activity against cells presenting said antigenic peptide as a foreign substance.

As described above, since T cell plays a central role in the immune system, a T cell therapy has been developed which is an approach including introducing a TCR gene that recognizes antigen peptide derived from virus, tumor or the like, a chimeric antigen receptor (CAR) and the like into cells derived from a patient, or an allogeneic cell with the same or substantially the same HLA genotype, artificially producing a large amount of cancer antigen-specific T cells in vitro, and infusing same into the living body. However, endogenous TCR is present in the T cells undergoing gene transfer, and the endogenous TCR competes with the introduced TCR to bind to the CD3 molecule required for TCR expression on the cell surface, which in turn causes a problem that the expression of the introduced TCR is inhibited. In addition, a problem of mispairing of the transgenic TCR chain with the endogenous TCR chain has also been pointed out. Furthermore, when allogeneic cells are used, there is also a possibility that the endogenous TCR recognizes the recipient's antigen and causes graft-versus-host disease (GvHD).

As a method for solving these problems, a method of introducing a single-stranded chimeric TCR in which a constant region of the TCRβ chain (Cβ is fused with a variable region of the α chain (Vα) and a variable region of the β chain (Vβ), and the constant region of TCRα chain (Cα) into cells has been reported. It has been reported that, using the method, a mismatch with the endogenous TCRα chain is considered to be suppressed, and thus an unexpected in vivo action can be suppressed (non-patent document 1). It has also been reported that the mismatch between the introduced TCR chain and the endogenous TCR chain can be suppressed by introducing cysteines into the constant region of the α chain and β chain of the TCR to be introduced (non-patent document 2). However, these documents focus primarily on preventing a mismatch between the introduced TCR chain and the endogenous TCR chain, have not verified the alloreactivity of the endogenous TCR chain, and do not disclose or even suggest a variant of TCR that does not have any of the complementarity determining regions (CDRs) of TCRα chain and β chain that are important for the recognition of an antigen-HLA complex.

### [Document List]

### [Non-patent documents]

non-patent document 1: Knies D. et al., Oncotarget, 7(16):21199-21221 (2016)
non-patent document 2: Kuball J. et al., blood, 109(6):2331-2338 (2007)

### [Summary of Invention]

### [Technical Problem]

Therefore, the problem of the present invention is to provide a novel T-cell receptor (TCR) variant that does not contain any of the complementarity determining regions (CDR) of the T cell receptor α chain and β chain. In addition, the problem includes provision of a T cell that expresses the variant and shows suppressed alloreactivity.

### [Solution to Problem]

During the development of a T cell receptor (TCR) with suppressed alloreactivity that can solve the above-mentioned problems, the present inventors have surprisingly found that alloreactivity, which is considered to be caused by endogenous TCR, is suppressed in the cells into which a T cell receptor variant not containing CDR of TCRα chain or β chain has been introduced. In addition, they have found that a modified TCR that combines different chains of the TCR chain, i.e., constant region of αβTCR and CDR of γδTCR, can similarly suppress alloreactivity of T cells. They have conducted intensive studies based on these findings, and completed the present invention.

Accordingly, the present invention provides the following.
[1] A variant of a T-cell receptor comprising a combination of two polypeptides comprising a constant region of a T cell receptor chain selected from the group consisting of α chain, β chain, γ chain and δ chain, wherein the polypeptide does not comprise a complementarity determining region (CDR) of the T cell receptor chain, a complementarity determining region (CDR) of the α chain, and a complementarity determining region (CDR) of the β chain.
[2] The variant of [1], wherein one of the aforementioned polypeptides comprises a constant region of the T cell receptor α chain or β chain, and the other comprises a constant region of the T cell receptor α chain or β chain.
[2a] The variant of [2], wherein at least one of the aforementioned polypeptides comprises a complementarity determining region (CDR) of the T cell receptor γ chain, and/or a complementarity determining region (CDR) of the T cell receptor δ chain.
[2b] The variant of [2a], wherein the constant region in at least one of the aforementioned polypeptides is a constant region of the α chain, and the complementarity determining region (CDR) is a complementarity determining region (CDR) of the γ chain.
[2c] The variant of [2a] or [2b], wherein the constant region in at least one of the aforementioned polypeptides is a constant region of the β chain, and the complementarity determining region (CDR) is a complementarity determining region (CDR) of the δ chain.
[3] The variant of any of [1] to [2c], wherein the two polypeptides are bound by one or more disulfide bonds.
[3a] The variant of any of [1] to [3], wherein the aforementioned polypeptide further comprises one or more signal peptides.
[3b] The variant of [3a], wherein the aforementioned signal peptide binds to the N-terminus of the constant region of the T cell receptor chain.
[3c] The variant of [3a], wherein the aforementioned signal peptide is a signal peptide of CD8 and/or IGH.
[4] A nucleic acid molecule encoding the variant of any of [1] to [3c].
[5] A vector comprising the nuclei acid molecule of [4].
[6] A method for producing a cell, comprising a step of introducing the vector of [5].
[6a] A pluripotent stem cell comprising a nucleic acid encoding the variant of any of [1] to [3c].
[6b] The cell of [6a], wherein the aforementioned pluripotent stem cell is an induced pluripotent stem cell (iPS cell).
[7] A cell expressing the variant of any of [1] to [3c].

### [Advantageous Effects of Invention]

When introduced into a cell, the variant of T-cell receptor of the present invention can suppress alloreactivity of the cell, and thus can reduce the risk of graft-versus-host disease (GvHD) in allogeneic transplantation.

### [Brief Description of Drawings]

Fig. 1 shows the detection results by flow cytometry of the expression of a CD3 molecule on the surface of a cell membrane of T cells expressing a variant of TCR which does not contain a variable region but contains the C region of the TCR chain. The horizontal axis shows expression of the CD3 molecule on cell membrane surface, and the vertical axis shows the number of cells.
Fig. 2 shows the measurement results by flow cytometry of the expression of CD3, CD5, CD7 and αβ TCRs on the cell membrane differentiated from an iPS cell transfected with AB6 by using a lentivirus vector.

### [Description of Embodiments]

### (Detailed Description of the Invention)

### 1. Variant of T-cell receptor

The present invention provides a variant of a T cell receptor comprising a combination of two polypeptides containing the constant region of a T cell receptor (TCR) chain selected from the group consisting of α chain, β chain, γ chain and δ chain (hereinafter sometimes to be referred to as "the variant of the present invention"). The variant of the present invention is characterized in that it does not include a complementarity determining region (CDR) of TCRα chain and β chain, or a complementarity determining region (CDR) of the same kind of chain (preferably a part or all of the variable region including CDR), and that it does not include a complementarity determining region (CDR) of T cell receptor chain from which the constant region derives, or a complementarity determining region (CDR) of the same kind of chain (preferably a part or all of the variable region including CDR). Accordingly, the variant of the present invention does not include a natural type or artificial type (e.g., the animal species from which constant region is derived and the animal species from which variable region is derived are different) of αβTCR, γδTCR, or TCR variants in which amino acids are further added to TCR of these. For example, when a polypeptide corresponding to at least one of the chains of the variant of the present invention contains the constant region of the T cell receptor α chain, the polypeptide does not contain the CDR of the T cell receptor α chain, preferably a part or all of the variable region containing the CDR. However, it may contain the CDR or variable region of TCR chain other than α chain and β chain, for example, γ chain or δ chain. Similarly, when a polypeptide corresponding to at least one of the chains of the above-mentioned variant contains the constant region of the T cell receptor β chain, the polypeptide does not contain the CDR of the T cell receptor β chain, preferably a part or all of the variable region containing the CDR. However, it may contain the CDR or variable region of TCR chain other than α chain and β chain, for example, γ chain or δ chain. The same applies to γ chain and δ chain. In one embodiment of the present invention, the T cell receptor β chain includes a T cell receptor β1 chain (SEQ ID NO: 2) or T cell receptor β2 chain (SEQ ID NO: 3). In one embodiment of the present invention, the T cell receptor β chain includes a T cell receptor β1 chain (SEQ ID NO: 2) and a T cell receptor β2 chain (SEQ ID NO: 3).

A membrane transfer signal peptide (hereinafter to be referred to as "signal peptide") may be further added to the polypeptide of the present invention. As the signal peptide, CD8, Immunoglobulin-H (IGH), CD4, a membrane localization signal peptide derived from a gene encoding various peptides having a transmembrane domain, and/or a signal peptide comprising an amino acid sequence of the signal peptide in which one or several (e.g., 2, 3, 4, 5) amino acids are deleted, substituted, inserted and/or added, or an amino acid sequence having identity with the amino acid sequence of the signal peptide can be used. When a signal peptide is added, the binding position and the number of signal peptides are not particularly limited.

In the present specification, "T cell receptor (TCR)" means a receptor constituted of a dimer of TCR chains (α chain, β chain, γ chain, δ chain), recognizes the antigen or the antigen-HLA (human leukocyte antigen) (MHC; major histocompatibility complex) complex and transmits a stimulation signal to the T cells. Each TCR chain is constituted of a variable region and a constant region, and the variable region contains three complementarity determining regions (CDR1, CDR2, CDR3). The variant of TCR means a dimer of the TCR chain in which each polypeptide contains at least the constant region of the above-mentioned TCR chain.

In one embodiment of the present invention, a variant in which one of the polypeptides constituting the variant of the present invention contains the constant region (to be also referred to as C region) of the T cell receptor α chain or β chain, and the other contains the constant region of the T cell receptor α chain or β chain is provided. In the variant, one of the polypeptides preferably contains CDR of TCR γ chain, preferably a part or all of the variable region of the TCR γ chain containing the CDR, and/or CDR of TCR δ chain, preferably a part or all of the variable region of the TCR δ chain containing the CDR. When at least a part of such variable region is contained, it is preferable that the constant region of at least one of the polypeptides constituting the variant of the present invention is the constant region of TCR α chain or β chain, and the CDR is CDR of γ chain or δ chain. More preferably, the constant region of one of the polypeptides constituting the variant of the present invention is the constant region of TCR α chain, and the CDR is CDR of δ chain.

In one embodiment of the present invention, a variant not containing a part or all of the variable region containing CDR of the TCR chain but containing C region is provided. In the variant, for example, one of the polypeptides may contain the constant region of T cell receptor α chain or β chain, and the other may contain the constant region of T cell receptor α chain or β chain. In the variant, for example, one of the polypeptides may contain the constant region of T cell receptor γ chain or δ chain, and the other may contain the constant region of T cell receptor γ chain or δ chain. Also, in the variant, for example, one of the polypeptides may contain the constant region of T cell receptor α chain or β chain, and the other may contain the constant region of T cell receptor γ chain or δ chain.

As a polypeptide constituting the variant of the present invention, for example, a polypeptide containing a constant region of a TCRα chain consisting of the amino acid sequence shown in SEQ ID NO: 1, the amino acid sequence shown in SEQ ID NO: 1 in which one or several (e.g., 2, 3, 4, 5) amino acids are deleted, substituted, inserted and/or added, or an amino acid sequence having identity with the amino acid sequence shown in SEQ ID NO: 1 (hereinafter to be referred to as "polypeptide 1") can be mentioned. In addition, as a polypeptide constituting the variant of the present invention, for example, a polypeptide containing a constant region of a TCR β chain consisting of the amino acid sequence shown in SEQ ID NO: 2 or 3, the amino acid sequence shown in SEQ ID NO: 2 or 3 in which one or several (e.g., 2, 3, 4, 5) amino acids are deleted, substituted, inserted and/or added, or an amino acid sequence having identity with the amino acid sequence shown in SEQ ID NO: 2 or 3 (hereinafter to be respectively referred to as "polypeptide 2" and "polypeptide 3") can be mentioned. In a preferred embodiment of the present invention, the variant of the present invention consists of the aforementioned polypeptide 1 (constant region of TCR α chain) and polypeptide 2 (constant region of TCR β1 chain), or the aforementioned polypeptide 1 (constant region of TCR α chain) and polypeptide 3 (constant region of TCR β2 chain).

In one embodiment of the present invention, to achieve more efficient expression of a polypeptide not containing a part or all of the variable region containing CDR of the TCR chain, but containing C region (e.g., the aforementioned variant composed of polypeptide 1 and polypeptide 2, or the aforementioned polypeptide 1 and polypeptide 3) on a cell membrane surface, it is preferable to further add a signal peptide to at least any of the polypeptides. As the signal peptide, CD8 or IGH is preferred. When a signal peptide is added, the binding position is not particularly limited, and it is preferably added to the C-terminus or N-terminus, more preferably the N-terminus, of the polypeptide containing the C region of TCR. When a signal peptide is added, the number of signal peptides is not particularly limited, and it is preferable to add one or more signal peptides, preferably one signal peptide.

Examples of the signal peptide include a signal peptide consisting of the amino acid sequence shown in SEQ ID NO: 4 (CD8) or 5 (IGH), the amino acid sequence shown in SEQ ID NO: 4 or 5 in which one or several (e.g., 2, 3, 4, 5) amino acids are deleted, substituted, inserted and/or added, or an amino acid sequence having identity with the amino acid sequence shown in SEQ ID NO: 4 or 5 (hereinafter to be referred to as "signal peptide 4" and "signal peptide 5", respectively).

In a preferred embodiment of the present invention, the variant of the present invention consists of the polypeptide shown in SEQ ID NO: 8 or 11 in which the aforementioned signal peptide 5 is added to the N-terminus of polypeptide 1 (hereinafter to be referred to as "polypeptide 8" or " polypeptide 11"), and the polypeptide shown in SEQ ID NO: 7 or 10 in which the aforementioned signal peptide 4 is added to the N-terminus of polypeptide 2 (hereinafter to be referred to as "polypeptide 7" or " polypeptide 10").

In another preferred embodiment of the present invention, it consists of polypeptide 8 or polypeptide 11, and the aforementioned polypeptide shown in SEQ ID NO: 13 or 15 in which the aforementioned signal peptide 4 is added to the N-terminus of polypeptide 3 (hereinafter to be referred to as "polypeptide 13" or "polypeptide 15").

As the polypeptide constituting the variant of the present invention, a polypeptide containing the variable region of the TCR γ chain can be mentioned. In addition, as the polypeptide constituting the variant of the present invention, a polypeptide containing the variable region of the TCR δ chain can be mentioned.

In the present specification, the "identity" means not less than 90% (e.g., 91%, 92%, 93%, 94%, 95%, 96%, 97%, 98% or 99% or still higher) identity. The identity of an amino acid sequence can be calculated using homology calculation algorithm NCBI BLAST (National Center for Biotechnology Information Basic Local Alignment Search Tool) (https://blast.ncbi.nlm.nih.gov/Blast.cgi) under the following conditions (expectancy=10; gap allowed; matrix=BLOSUM62; filtering=OFF). To determine the identity, it is appreciated that the full-length sequence of the present invention is compared with other sequences. In other words, the identity in the present invention excludes comparing a short fragment of the sequence of the present invention (e.g., 1-3 amino acids) with other sequence, or vice versa.

The derivation of the constant region, variable region and CDR of the TCR chain contained in the variant of the present invention is not particularly limited, and they are preferably derived from animal mammals (e.g., mouse, rat, hamster, rabbit, cat, dog, bovine, sheep, monkey, human), more preferably human.

In addition, the constant region of the TCR chain contained in the variant of the present invention is preferably subjected to specified modification in the constant region of the natural TCR chain. Examples of this modification include, but are not limited to, enhancement of a dimer expression efficiency due to a disulfide bond between the α chain and the β chain by substituting a particular amino acid residue in the constant region of the natural TCR with a cysteine residue (e.g., substitution of the 48th threonine in the constant region consisting of the amino acid sequence shown in SEQ ID NO: 1 with cysteine, substitution of the 56th or 55th serine in the constant region consisting of the amino acid sequence shown in SEQ ID NO: 2 or SEQ ID NO: 3 with cysteine). In a preferred embodiment, two polypeptides are preferably bonded by one or more, preferably two or more, disulfide bonds. The disulfide bond is formed by oxidation or post-translational modification between the cysteine residues (which may be contained in the natural type or artificially introduced as described above) contained in each polypeptide of the variant.

The variant of the present invention can be genetically produced using the nucleic acid or the vector of the present invention described later. For example, it can be produced by introducing both a nucleic acid encoding one of the polypeptides constituting the variant of the present invention and a nucleic acid encoding the other polypeptide into a cell to express respective polypeptides and form a dimer, and isolating the dimer by a method known per se.

### 2. Nucleic acid encoding the variant of the present invention or vector containing the nucleic acid

The present invention provides a nucleic acid encoding the aforementioned TCR of the present invention (hereinafter sometimes to be referred to as "the nucleic acid of the present invention"). As the nucleic acid of the present invention, the nucleic acid encoding one of the polypeptides constituting the variant of the present invention and the nucleic acid encoding the other polypeptide may be contained in different molecules, or the both nucleic acids encoding the polypeptide may be contained in a single molecule.

The nucleic acid of the present invention may be DNA or RNA, or DNA/RNA chimera, and preferably DNA. In addition, the nucleic acid may be double-stranded or single-stranded. In the case of double strands, a double-stranded DNA, a double-stranded RNA or a DNA:RNA hybrid may be used. When the nucleic acid is an RNA, T in the Sequence Listing is to be read as U as regards the RNA sequence. In addition, the nucleic acid of the present invention may contain a natural nucleotide, a modified nucleotide, a nucleotide analogue, or a mixture of these as long as it can express the polypeptide in vitro or in a cell.

The nucleic acid in the present invention can be produced by a method known per se and, for example, oligo DNA primers are synthesized to cover the desired portion of the sequence based on the known DNA sequence information of the TCR chain and, using the total RNA or mRNA fraction prepared from the cells having the sequence as the template, the nucleic acid can be cloned by amplification by the RT-PCR method. Alternatively, a DNA strand is chemically synthesized, or synthesized partially overlapping oligo DNA short chains are connected using a PCR method (overlap PCR method) or a Gibson Assembly method, whereby a DNA encoding the full length or a part of the nucleic acid can be constructed.

The nucleic acid of the present invention can be incorporated into an expression vector. Therefore, the present invention provides an expression vector containing the aforementioned nucleic acid of the present invention (hereinafter sometimes to be referred to as "the vector of the present invention").

Examples of the promoter to be used in the vector of the present invention include ubiquitin promoter, EF1α promoter, CAG promoter, SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (simple herpes virus thymidine kinase) promoter and the like. Of these, ubiquitin promoter, EF1α promoter, CAG promoter, MoMuLV LTR, CMV promoter, SRα promoter and the like are preferable.

The vector of the present invention may contain a transcription and translation regulatory sequence, a ribosome binding site, an enhancer, a replication origin, a polyA addition signal, a selection marker gene and the like on demand besides the above-mentioned promoters. Examples of the selection marker gene include dihydrofolate reductase gene, neomycin resistance gene, puromycin resistance gene and the like.

In one embodiment of the present invention, heterodimers of both polypeptides can be constructed in a cell or on the cell surface by introducing an expression vector containing a nucleic acid encoding one of the polypeptides constituting the aforementioned variant of the present invention and a nucleic acid encoding the other polypeptide into the target cell. In this case, the nucleic acid encoding one of the polypeptides constituting the aforementioned variant of the present invention and the nucleic acid encoding the other polypeptide may be incorporated into separate expression vectors or a single expression vector. When they are incorporated into a single expression vector, these two kinds of nucleic acids are preferably incorporated via a sequence enabling polycistronic expression. Using a sequence enabling polycistronic expression, plural genes incorporated in one kind of expression vector can be more efficiently expressed. Examples of the sequence enabling polycistronic expression include 2A sequence (e.g., 2A sequence derived from foot-and-mouth disease virus (FMDV) (F2A), 2A sequence derived from horse rhinitis Avirus (ERAV) (E2A), 2A sequence derived from Porcineteschovirus (PTV-1) (P2A), 2A sequence derived from Thosea asigna virus (TaV) (T2A)) (PLoS ONE, 3, e2532, 2008, Stem Cells 25, 1707, 2007, etc.), internal ribosome entry site (IRES) (U.S. Patent No. 4,937,190) and the like. From the aspect of uniform expression levels, 2A sequence is preferable. Among the 2A sequences, P2A sequence and T2A sequence are preferred.

The expression vector that can be used in the present invention includes viral vector, plasmid vector and the like. As the virus vector, retrovirus vector (including lentivirus vector and pseudo type vector), adenovirus vector, adeno-associated virus vector, herpes virus vector, Sendaivirus, episomal vector and the like can be mentioned. A transposon expression system (e.g., PiggyBac system) may also be used. As the plasmid vector, animal cell expression plasmid (e.g., pa1-11, pXT1, pRc/CMV, pRc/RSV, pcDNAI/Neo) and the like can be mentioned.

### 3. T cell expressing the variant of the present invention

The present invention provides a cell comprising the nucleic acid or the vector of the present invention introduced thereinto (hereinafter sometimes to be referred to as "the cell of the present invention"). The cell of the present invention preferably expresses the variant of the present invention.

As a cell into which the nucleic acid or the expression vector of the present invention is introduced, for example, lymphocytes and progenitor cells of lymphocytes, and pluripotent stem cells can be mentioned. In the present invention, the "lymphocyte" means one of the subtypes of leukocytes in the immune system of vertebrata. As the lymphocyte, T-cells, B-cells and Natural killer cells can be mentioned. As the cell into which the nucleic acid or the vector of the present invention is introduced, pluripotent stem cell is preferred.

In the present invention, the "T cell" means a CD3 positive cell. Examples of the T cell that expresses the variant of the present invention include cytotoxic T lymphocyte (CTL) which is a CD8 positive cell, helper T cell which is a CD4 positive cell, regulatory T cell, effector T cell and the like, with preference given to the cytotoxic T lymphocyte. T cell that expresses the variant of the present invention can be obtained by introducing the nucleic acid or the vector of the present invention into T cells collected from a living body. Alternatively, T cells expressing the TCR of the present invention can be obtained by inducing differentiation from pluripotent stem cells or lymphocyte progenitor cells into T cells into which the nucleic acid or the vector of the present invention has been introduced (namely, T cells derived from the pluripotency cells or the progenitor cells).

In one embodiment of the present invention, T cell sometimes expresses CD5 and/or CD7 as well as CD3. CD5 and/or CD7 may also be expressed on the cell surface of T cells in the living body. When T cell expresses CD5 and/or CD7, CD5 and CD7 are expressed without forming a complex with the TCR molecule, whereas CD3 is expressed on the cell surface by forming a complex with the TCR in the T cell.

The cell of the present invention (e.g., cytotoxic T cell) also has, in addition to the TCR gene inherently present in the cell, an exogenous TCR gene derived from the nucleic acid or the vector of the present invention. On this point, the cell of the present invention is different from the cells harvested from the living body. The cell of the present invention may express chimeric antigen receptor (CAR) as well as the variant of the present invention.

The aforementioned lymphocytes can be collected from, for example, peripheral blood, bone marrow and cord blood of a human or a non-human mammal. When a cell that expresses the variant of the present invention is used for the treatment of diseases such as cancer, the cell population is preferably collected from the subject to be treated or from a donor matched with the HLA type of the treatment target.

Examples of the progenitor cell of lymphocytes include hematopoietic stem cell, multipotent progenitor cell without self-replication competence (MMP), myelo-lymphoid progenitor (MLP) cell, myeloid progenitor (MP) cell, granulo-monocyte progenitor (GMP) cell, macrophage-dendritic cell progenitor (MDP) cell, dendritic cell precursor (DCP) cell and the like. Examples of the pluripotent stem cell include embryonic stem cell (ES cell), induced pluripotent stem cell (iPS cell), embryonal carcinoma cell (EC cell), embryonic germ cell (EG cell) and the like. When the above-mentioned pluripotent stem cell is ES cell or any cell derived from human embryo, the cell may be a cell produced by destroying the embryo or a cell prepared without destroying the embryo. Preferred is a cell produced without destroying the embryo.

In the present specification, the "pluripotent stem cell" refers to an embryonic stem cell (ES cell) and a cell inherently having differentiation pluripotency similar thereto, namely, the ability to differentiate into various tissues (all of endoderm, mesoderm, ectoderm) in a living body. As the cell having differentiation pluripotency similar to that of ES cell, an "induced pluripotent stem cell" (sometimes to be referred to as "iPS cell" in the present specification) can be mentioned.

As the "ES cell", various mouse ES cell lines established by inGenious targeting laboratory, RIKEN (Inst. of Physical and Chemical Research) and the like can be used as the mouse ES cell, and various human ES cell lines established by NIH, RIKEN, Kyoto University, Cellartis and the like can be used. For example, as the human ES cell line, CHB-1 - CHB-12 strains, RUES1 strain, RUES2 strain, HUES1 - HUES28 strains and the like of NIH, H1 strain, H9 strain and the like of WisCell Research, KhES-1 strain, KhES-2 strain, KhES-3 strain, KhES-4 strain, KhES-5 strain, SSES1 strain, SSES2 strain, SSES3 strain and the like of RIKEN can be used.

In the present specification, the "induced pluripotent stem cell (iPS cell)" refers to a cell obtained by introducing a specific factor (nuclear reprogramming factor) into a mammalian somatic cell or an undifferentiated stem cell and reprogramming them. Currently, there are various types of the "induced pluripotent stem cell", and iPS cell established by Yamanaka, et al. by introducing 4 factors of Oct3/4, Sox2, Klf4, c-Myc into mouse fibroblast (Takahashi K, Yamanaka S., Cell, (2006) 126: 663-676), iPS cell derived from a human cell established by introducing similar 4 factors into human fibroblast (Takahashi K, Yamanaka S., et al., Cell, (2007) 131: 861-872.), Nanog-iPS cell established by selecting with the expression of Nanog as an index after introduction of the above-mentioned 4 factors (Okita, K., Ichisaka, T., and Yamanaka, S. (2007). Nature 448, 313-317.), iPS cell produced by a method free of c-Myc (Nakagawa M, Yamanaka S., et al., Nature Biotechnology, (2008) 26, 101 - 106), and iPS cell established by introducing 6 factors by a virus-free method (Okita K et al., Nat. Methods 2011 May; 8(5):409-12, Okita K et al., Stem Cells. 31(3):458-66.) can also be used. In addition, induced pluripotent stem cell established by Thomson et al. by introducing 4 factors of OCT3/4, SOX2, NANOG, and LIN28 (Yu J., Thomson JA. et al., Science (2007) 318: 1917-1920.), induced pluripotent stem cell produced by Daley et al. (Park IH, Daley GQ. et al., Nature (2007) 451: 141-146), induced pluripotent stem cell produced by et al. (JP-A-2008-307007) and the like can also be used.

In addition, any of the induced pluripotent stem cells known in the art that are described in published papers (e.g., Shi Y., Ding S., et al., Cell Stem Cell, (2008) Vol3, Issue 5,568-574; Kim JB., Scholer HR., et al., Nature, (2008) 454, 646-650; Huangfu D., Melton, DA., et al., Nature Biotechnology, (2008) 26, No 7, 795-797), or patents (e.g., JP-A-2008-307007, JP-A-2008-283972, US2008-2336610, US2009-047263, WO2007/069666, WO2008/118220, WO2008/124133, WO2008/151058, WO2009/006930, WO2009/006997, WO2009/007852) can be used.

As the induced pluripotent stem cell line, various iPS cell lines established by NIH, RIKEN, Kyoto University and the like can be used. Examples of the human iPS cell line include HiPS-RIKEN-1A strain, HiPS-RIKEN-2A strain, HiPS-RIKEN-12A strain, Nips-B2 strain of RIKEN, 253G1 strain, 201B7 strain, 409B2 strain, 454E2 strain, 606A1 strain, 610B1 strain, 648A1 strain, 1231A3 strain, Ff-I01s04 strain, and the like.

In the present invention, the "hematopoietic progenitor cell" is a pluripotent stem cell (multipotent stem cell) capable of differentiating into hematopoietic cells. In humans, it is primarily present in the bone marrow and differentiates into leukocyte (neutrophil, eosinophil, basophil, lymphocyte, monocyte, macrophage), erythrocyte, platelet, mast cell, and dendritic cell. In the present invention, the "hematopoietic progenitor cell" means a CD34 positive cell, preferably, a CD34/CD43 double positive (DP) cell. The derivation of the hematopoietic progenitor cell to be used in the present invention is not particularly limited and may be obtained by, for example, inducing differentiation of a pluripotent stem cell by the method described below, or isolated from a biological tissue by a known method.

When the nucleic acid or expression vector of the present invention is introduced into a pluripotent stem cell or a progenitor cell of lymphocyte, the cell is preferably differentiated into lymphocyte, preferably T cell, by a method known per se. As a method for differentiating pluripotent stem cells into T cells, for example, a method including (1) a step of differentiating pluripotent stem cells comprising the nucleic acid or the vector of the present invention into hematopoietic progenitor cells, and (2) a step of differentiating the hematopoietic progenitor cells into T cells can be mentioned. As the aforementioned step (1), for example, a method including culturing pluripotent stem cells in a medium for induction of hematopoietic progenitor cells, as described in, for example, WO 2013/075222, WO 2016/076415 and Liu S. et al., Cytotherapy, 17 (2015); 344-358 and the like can be mentioned. As the aforementioned step (2), a method containing (2-1) a step of inducing CD4CD8 double positive T cells from the hematopoietic progenitor cells and (2-2) a step of inducing CD8 positive T cells from the CD4CD8 double positive T cells, as described in e.g. WO 2016/076415 and the like can be mentioned.

There is no particular limitation on the method for introducing the nucleic acid or the vector of the present invention into cells, and known methods can be used. When the nucleic acid or the plasmid vector is introduced, for example, a calcium phosphate coprecipitation method, a PEG method, an electroporation method, a microinjection method, a lipofection method and the like can be used. For example, the methods described in Cell Engineering additional volume 8, New Cell Engineering experiment protocol, 263-267 (1995) (published by Shujunsha), Virology, vol. 52, 456 (1973), Folia Pharmacol. Jpn., vol. 119 (No. 6), 345-351 (2002) and the like can be used. When a virus vector is used, the nucleic acid of the present invention is introduced into a suitable packaging cell (e.g., Plat-E cell) and a complementation cell line (e.g., 293 cell), the virus vector produced in the culture supernatant is recovered, and cells are infected with the vector by an appropriate method suitable for each virus vector, whereby the vector is introduced into the cells. For example, when a retrovirus vector is used as the vector, a specific means is disclosed in WO 2007/69666, Cell, 126, 663-676 (2006) and Cell, 131, 861-872 (2007) and the like. In addition, a specific means for using lentivirus as the vector is disclosed in Zufferey R. et al., Nat Biotechnol, 15(9):871-895 (1997) and the like. Particularly, when a retrovirus vector is used, highly efficient transfection into various cells is possible by using a recombinant fibronectin fragment CH-296 (manufactured by Takara Bio Inc.). Alternatively, the nucleic acid or vector of the present invention HLA gene, B2M gene, and/or CIITA gene in the cells may be introduced into the cell genome by genome editing (e.g., CRISPR system, TALEN, ZFN and the like).

The nucleic acid of the present invention may also be used in direct introduction into cells in the form of an RNA for expressing the variant of the present invention in the cells. As a method for introducing the RNA, a known method can be used and, for example, a lipofection method, an electroporation method, or the like can be preferably used.

The expression of the variant of the present invention can be detected or measured by, for example, an immunological method using an antibody capable of recognizing a part of the variant of the present invention (e.g., constant region of TCR chain, etc.). Examples of the immunological method include antibody array, flow cytometric analysis, radioisotopic immunoassay method (RIA method), ELISA, Western blotting, immunohistostaining, enzyme immunoassay (EIA method), fluorescent immunoassay (FIA), immunochromatography method and the like.

That the variant of the present invention suppresses alloreactivity of cells can be confirmed by a method known per se. For example, a cell expressing the variant of the present invention and a target cell not expressing the variant are subjected to a mixed leukocyte reaction (MLR), an Elispot assay, a limiting dilution assay, or the like, and when at least one of the assays results in low alloreactivity, it can be evaluated that the alloreactivity was suppressed by the variant of the present invention.

### 5. Production method of the cell of the present invention

The present invention also provides a production method of a cell, including a step of introducing the nucleic acid or the vector of the present invention into a cell (hereinafter sometimes to be referred to as "the production method of the present invention"). The cell into which the nucleic acid or the vector of the present invention is introduced, the introduction method and the like are as described in the above-mentioned 3. The aforementioned cell preferably expresses the variant of the present invention. The expression of the variant of the present invention can be detected or measured by the method described in the above-mentioned 3.

In one embodiment of the production method of the present invention, a production method of a T cell including (1) a step of differentiating pluripotent stem cells comprising the nucleic acid or the vector of the present invention into hematopoietic progenitor cells, and (2) a step of differentiating the hematopoietic progenitor cells into T cells is provided.

### (1) Step of differentiating pluripotent stem cells into hematopoietic progenitor cells (step (1))

The method of differentiating pluripotent stem cells into hematopoietic progenitor cells is not particularly limited as long as it can cause differentiation into hematopoietic progenitor cells. Examples thereof include a method including culturing pluripotent stem cells in a medium for induction of hematopoietic progenitor cells, as described in, for example, WO 2013/075222, WO 2016/076415 and Liu S. et al., Cytotherapy, 17 (2015); 344-358 and the like.

In the present invention, a medium used for induction into hematopoietic progenitor cells is not particularly limited. A medium used for culturing animal cells can be prepared into a basal medium. Examples of the basal medium include Iscove's Modified Dulbecco's Medium (IMDM), Medium 199, Eagle's Minimum Essential Medium (EMEM), αMEM medium, Dulbecco's modified Eagle's Medium (DMEM), Ham's F12 medium, RPMI 1640 medium, Fischer's medium, and Neurobasal Medium (Life Technologies), and a mixed medium of these. The medium may contain a serum, or may be serum-free. If necessary, the basal medium may also contain Vitamin Cs (e.g., ascorbic acid), albumin, insulin, transferrin, selenium, fatty acid, trace elements, 2-mercaptoethanol, thiol glycerol, lipids, amino acids, L-glutamine, non-essential amino acids, vitamins, growth factors, low-molecular-weight compounds, antibiotics, antioxidants, pyruvic acid, buffers, inorganic salts, cytokines, and the like.

In the present invention, the "Vitamin Cs" means L-ascorbic acid and derivatives thereof, and "L-ascorbic acid derivative" means derivatives that become vitamin C by enzymatic reaction in the living body. Examples of the derivatives of L-ascorbic acid to be used in the present invention include vitamin C phosphate, ascorbic acid glucoside, ascorbyl ethyl, vitamin C ester, ascorbyl tetrahexyldecanoate, ascorbyl stearate, and ascorbyl 2-phosphate 6-palmitate. Preferred is vitamin C phosphate. Examples of the vitamin C phosphate include salts of L-ascorbic acid phosphate such as L-ascorbic acid phosphate Na and L-ascorbic acid phosphate Mg.

The basal medium to be used in step (1) is preferably IMDM medium containing serum, insulin, transferrin, selenium, thiol glycerol, L-glutamine and ascorbic acid.

The medium to be used in step (1) may be further supplemented with at least one kind of cytokine selected from the group consisting of BMP4 (Bone morphogenetic protein 4), VEGF (vascular endothelial growth factor), SCF (Stem cell factor), and FLT-3L (Flt3 Ligand). The medium is more preferably a culture liquid supplemented with VEGF, SCF and FLT-3L.

When Vitamin Cs is used in step (1), the Vitamin Cs is preferably added (supplied) every four days, every three days, every two days, or every day. The Vitamin Cs is preferably added every day. The concentration of the vitamin Cs in the medium is not particularly limited but is preferably an amount corresponding to 5 ng/ml to 500 ng/ml (e.g., an amount corresponding to 5 ng/ml, 10 ng/ml, 25 ng/ml, 50 ng/ml, 100 ng/ml, 200 ng/ml, 300 ng/ml, 400 ng/ml, or 500 ng/ml).

When BMP4 is used in step (1), the concentration of the BMP4 in the medium is not particularly limited. It is preferably 10 ng/ml - 100 ng/ml (e.g., 10 ng/ml, 20 ng/ml, 30 ng/ml, 40 ng/ml, 50 ng/ml, 60 ng/ml, 70 ng/ml, 80 ng/ml, 90 ng/ml, 100 ng/ml), more preferably 20 ng/ml - 40 ng/ml.

When VEGF is used in step (1), the concentration of the VEGF in the medium is not particularly limited. It is preferably 10 ng/ml - 100 ng/ml (e.g., 10 ng/ml, 20 ng/ml, 30 ng/ml, 40 ng/ml, 50 ng/ml, 60 ng/ml, 70 ng/ml, 80 ng/ml, 90 ng/ml, 100 ng/ml), particularly preferably 20 ng/ml.

When SCF is used in step (1), the concentration of the SCF in the medium is not particularly limited. It is preferably 10 ng/ml - 100 ng/ml (e.g., 10 ng/ml, 20 ng/ml, 30 ng/ml, 40 ng/ml, 50 ng/ml, 60 ng/ml, 70 ng/ml, 80 ng/ml, 90 ng/ml, 100 ng/ml), particularly preferably 30 ng/ml.

When FLT-3L is used in step (1), the concentration of the FLT-3L in the medium is not particularly limited. It is preferably 1 ng/ml - 100 ng/ml (e.g., 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 20 ng/ml, 50 ng/ml, 100 ng/ml), particularly preferably 10 ng/ml.

In step (1), the pluripotent stem cells may be cultured by adherent culture or suspension culture. In cases of the adherent culture, the culture may be carried out in a culture vessel coated with a coating agent, and/or may be co-cultured with other cells. Examples of the other cells for the co-culture include C3H10T1/2 (Takayama N., et al. J Exp Med. 2817-2830, 2010) and stromal cells derived from a different species (Niwa A et al. J Cell Physiol. 2009 Nov; 221(2): 367-77). Examples of the coating agent include Matrigel (Nivea A, et al. PLoS One. 6(7): e22261, 2011). Examples of the method of the suspension culture include the methods described in Chadwick et al. Blood 2003, 102: 906-15, Vijayaragavan et al. Cell Stem Cell 2009, 4: 248-62, and Saeki et al. Stem Cells 2009, 27: 59-67.

In step (1), the temperature conditions are not limited. The temperature is, for example, about 37°C to about 42°C, preferably about 37 to about 39°C. The culture period may be appropriately determined by those skilled in the art by monitoring the number of hematopoietic progenitor cells and the like. The number of days of the culture is not limited as long as hematopoietic progenitor cells can be obtained. Examples of the culture period include at least 6 days, not less than 7 days, not less than 8 days, not less than 9 days, not less than 10 days, not less than 11 days, not less than 12 days, not less than 13 days, and not less than 14 days. The culture period is preferably 14 days. While a longer culture period generally does not pose a problem in the production of hematopoietic progenitor cells, it is preferably not more than 35 days, more preferably not more than 21 days. The culture may be carried out under low-oxygen conditions, and the low-oxygen condition in the present invention means, for example, oxygen concentration of 15%, 10%, 9%, 8%, 7%, 6%, 5% or lower than these.

### (2) Step of differentiating the hematopoietic progenitor cells into T cells (step (2))

A method for differentiating the hematopoietic progenitor cells into T cells is not particularly limited as long as it can differentiate hematopoietic progenitor cells into T cells. Examples thereof include a method containing (2-1) a step of inducing CD4CD8 double positive T cells from the hematopoietic progenitor cells and (2-2) a step of inducing CD8 positive T cells from the CD4CD8 double positive T cells, as described in e.g. WO 2016/076415 and the like. It is preferable to isolate the hematopoiesis precursor in advance from the cell population obtained in step (1) by using a marker of a hematopoietic progenitor cell. As the marker, at least one selected from the group consisting of CD43, CD34, CD31 and CD144 can be mentioned.

### (2-1) Step of inducing the hematopoietic progenitor cell into the CD4CD8 double positive T cell (step (2-1))

In the present invention, examples of the differentiation method into the CD4CD8 double positive T cell include a method of culturing the hematopoietic progenitor cell in an induction medium into the CD4CD8 double positive T cell.

In the present invention, a medium for inducing differentiation into the CD4CD8 double positive T cell is not particularly limited, and a medium used for culturing animal cells can be prepared into a basal medium. Examples of the basal medium include those similar to the basal medium used in the above-mentioned step (1). The medium may contain serum, or may be serum-free. If necessary, the basal medium may also contain Vitamin Cs, albumin, insulin, transferrin, selenium, fatty acid, trace elements, 2-mercaptoethanol, thiol glycerol, lipids, amino acids, L-glutamine, non-essential amino acids, vitamins, growth factors, low-molecular-weight compounds, antibiotics, antioxidants, pyruvic acid, buffers, inorganic salts, cytokines, and the like.

A preferable basal medium to be used in step (2-1) is αMEM medium containing serum, transferrin, selenium and L-glutamine. When Vitamin Cs is added to the basal medium, Vitamin Cs is the same as that in step (1).

The medium used in step (2-1) may further contain cytokine FLT-3L and/or IL-7, more preferred is a culture medium containing FLT-3L and IL-7.

When IL-7 is used in step (2-1), the concentration of the IL-7 in the culture medium is preferably 1 ng/ml - 50 ng/ml (e.g., 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 20 ng/ml, 30 ng/ml, 40 ng/ml, 50 ng/ml), particularly preferably 5 ng/ml.

When FLT-3L is used in step (2-1), FLT-3L can be used similarly to the above-mentioned step (1).

In step (2-1), the hematopoietic progenitor cells may be cultured by adherent culture or suspension culture. In cases of the adherent culture, a coated culture vessel may be used, and/or the hematopoietic progenitor cells may be co-cultured with feeder cells and the like. Examples of the feeder cell for the co-culture include a bone-marrow stromal cell line, OP9 cell (available from Riken BioResource Center). The OP9 cell is preferably OP9-DL4 cell or OP9-DL1 cell, which constantly expresses DLL4 or DLL1 (Holmes R I and Zuniga-Pflucker J C. Cold Spring Harb Protoc. 2009). In the present invention, in cases where OP9 cells are used as the feeder cells, DLL4 or DLL1, or a fusion protein of DLL4 or DLL1 and Fc or the like, separately prepared may be added to the medium. When feeder cell is used, the feeder cells are preferably appropriately replaced during the culture. The replacement of the feeder cells may be carried out by transferring the subject cells that are being cultured onto feeder cells that are preliminarily plated. The replacement may be carried out every five days, every four days, every three days, or every two days. When hematopoietic progenitor cells are obtained by suspension culture of embryoid, it is preferable to perform adhesion culture after dissociation into single cells. While the cells may be co-cultured with feeder cells, culturing is preferably carried out without using feeder cells.

In the case of adhesion culture and when a culture container is coated, examples of the coating agent include Matrigel (Niwa A, et al. PLos One, 6(7):e22261, 2011)), collagen, gelatin, laminin, heparan sulfuric acid proteoglycan, RetroNectin, DLL4 or DLL1, fusion protein of DLL4 or DLL1 and Fc region of antibody and the like (e.g., DLL4/Fc chimera), entactin, and/or combination of these, and a combination of RetroNectin and fusion protein of DLL4 and Fc region, etc. is preferable.

In step (2-1), the culture temperature conditions are not limited. The temperature is, for example, about 37°C to about 42°C, preferably about 37°C to about 39°C. The culture period may be appropriately determined by those skilled in the art by monitoring the number of CD4/CD8 double-positive T cells and the like. The number of days of the culture is not limited as long as hematopoietic progenitor cells can be obtained. Examples of the culture period include at least not less than 10 days, not less than 12 days, not less than 14 days, not less than 16 days, not less than 18 days, not less than 20 days, not less than 22 days, and not less than 23 days. The culture period is preferably 23 days. In addition, not more than 90 days is preferable, and not more than 42 days is more preferable.

### (2-2) Step of inducing CD8 positive T cells (CD3 single positive T cell) from the CD4CD8 double positive (DP) T cells (step (2-2))

The CD4/CD8 DPT cells obtained by step (2-1) can be induced to differentiate into CD8 single positive T cells by subjecting them to a step for inducing differentiation into CD8 positive T cells.

Examples of the basal medium and medium to be used in step (2-2) include those similar to the basal medium and medium used in step (1).

The aforementioned medium may contain an adrenocortical hormone agent. Examples of the adrenocortical hormone agent include, for example, a glucocorticoid and a derivative thereof. Examples of the glucocorticoid include, for example, cortisone acetate, hydrocortisone, fludrocortisone acetate, prednisolone, triamcinolone, methylprednisolone, dexamethasone, betamethasone, and beclometasone dipropionate. Of these, dexamethasone is preferable.

When dexamethasone is used as the adrenocortical hormone agent, the concentration of the dexamethasone in the medium is preferably 1 nM - 100 nM (e.g., 1 nM, 5 nM, 10 nM, 20 nM, 30 nM, 40 nM, 50 nM, 60 nM, 70 nM, 80 nM, 90 nM, 100 nM), particularly preferably 10 nM.

The aforementioned medium may contain an antibody (e.g., anti-CD3 antibody, anti CD28 antibody, anti CD2 antibody), or cytokine (e.g., IL-7, IL-2, IL-15) and the like.

When an anti-CD3 antibody is used in step (2-2), the anti-CD3 antibody is not particularly limited as long as it specifically recognizes CD3. For example, an antibody produced from OKT3 clone can be mentioned. The anti-CD3 antibody may be bonded to magnetic beads and the like or, instead of adding the aforementioned anti-CD3 antibody to the medium, stimulation may be given by incubating the T lymphocytes for a given period on a culture vessel to which the anti-CD3 antibody is bound on the surface thereof. The concentration of the anti-CD3 antibody in the medium is preferably 10 ng/ml - 1000 ng/ml (e.g., 10 ng/ml, 50 ng/ml, 100 ng/ml, 200 ng/ml, 300 ng/ml, 400 ng/ml, 500 ng/ml, 600 ng/ml, 700 ng/ml, 800 ng/ml, 900 ng/ml, 1000 ng/ml), particularly preferably 500 ng/ml. The concentration of other antibodies can also be appropriately determined by those of ordinary skill in the art based on the culture conditions and the like.

When IL-2 is used in step (2-2), the concentration of the IL-2 in the medium is preferably 10 U/ml - 1000 U/ml (e.g., 10 U/ml, 20 U/ml, 30 U/ml, 40 U/ml, 50 U/ml, 60 U/ml, 70 U/ml, 80 U/ml, 90 U/ml, 100 U/ml, 200 U/ml, 500 U/ml, 1000 U/ml), particularly preferably 100 U/ml. The concentration of the IL-7 or IL-15 in the medium used in step (2-2) is preferably 1 ng/ml - 100 ng/ml (e.g., 1 ng/ml, 5 ng/ml, 10 ng/ml, 20 ng/ml, 30 ng/ml, 40 ng/ml, 50 ng/ml, 60 ng/ml, 70 ng/ml, 80 ng/ml, 90 ng/ml, 100 ng/ml), particularly preferably 10 ng/ml.

In step (2-2), the temperature conditions are not particularly limited. The temperature is preferably about 37°C to about 42°C, more preferably about 37°C to about 39°C. The culture period may be appropriately determined by those skilled in the art by monitoring of the number of CD8-positive T cells and the like. The number of days of the culture is not limited as long as CD8-positive T cells can be obtained. The culture period is preferably not less than 1 day, not less than 3 days, not less than 7 days, and preferably not more than 60 days, more preferably not more than 35 days.

In another embodiment, the present invention provides a method for reducing alloreactivity of a cell, including a step of introducing the nucleic acid or vector of the present invention into the cell.

The present invention is explained in more detail in the following by referring to Examples, which are mere exemplifications and do not limit the present invention.

### [Example]

### [Example 1] Production of LentiV vector containing nucleic acid encoding variant of TCR having constant region of αβ TCR, and not having complementarity determining region (CDR)

A polypeptide chain that connects, using a P2A sequence, the amino acids of each α chain (TRAC) with a CD8 molecule membrane localization signal peptide added to its N-terminal, and each β chain (TRBC1 or TRBC2) with an IGH molecule membrane localization signal peptide added to its N-terminal was designed (Table 1 AB5-AB8). The oligo DNA encoding the designed polypeptide chain was artificially synthesized (GenScript) and inserted into the multicloning site of the lentivirus vector plasmid. In the lentivirus vector plasmid, the CMV promoter of pCDH-CMV-MCS-EF1a-Puro (SystemBioscience) was substituted with the human ubiquitin promoter. The production of the virus vector was outsourced to SIRION.

**[Table 1]**

| Vector No. | lead | TRBC | 2A | Lead | TRAC | base sequence contained in introduced gene | peptide sequence 1 (CD8-Cβ) to be expressed | peptide sequence 2 (IGH-Cα) to be expressed |
|---|---|---|---|---|---|---|---|---|
| AB5 | CD8 | TRBC1 | P2A | IGH | TRAC | SEQ ID NO: 6 | SEQ ID NO: 7 | SEQ ID NO: 8 |
| AB6 | CD8 | TRBC1 S56C | P2A | IGH | TRAC T48C | SEQ ID NO: 9 | SEQ ID NO: 10 | SEQ ID NO: 11 |
| AB7 | CD8 | TRBC2 | P2A | IGH | TRAC | SEQ ID NO: 12 | SEQ ID NO: 13 | SEQ ID NO: 8 |
| AB8 | CD8 | TRBC2 S55C | P2A | IGH | TRAC T48C | SEQ ID NO: 14 | SEQ ID NO: 15 | SEQ ID NO: 11 |

TRAC: SEQ ID NO: 1
   TRAC T48C: SEQ ID NO: 1 wherein 48th threonine is substituted with cysteine
TRBC1: SEQ ID NO: 2
   TRBC1 S56C: SEQ ID NO: 2 wherein 56th serine is substituted with cysteine
TRBC2: SEQ ID NO: 3
   TRBC2 S55C: SEQ ID NO: 3 wherein 55th serine is substituted with cysteine
CD8: SEQ ID NO: 4
IGH: SEQ ID NO: 5

### [Example 2] Production of T cell expressing TCR variant

Using a 24 well plate coated with RetroNectin (Takara Bio Inc.), K562 cells (K562-CD3 cells, supplied by Dr. Uemura, National Cancer Research Center) forcibly expressing four types of CD3 genes (γ, δ, ε and ζ) were infected with lentiviral vector carrying the gene encoding each variant shown in Table 1 to transfect the gene encoding each variant. After infection with the lentivirus vector, the cells were cultured under 37°C, 5% CO₂ conditions for 3 days.

### [Experimental Example 1] Evaluation of cell membrane surface molecule expression of T cell that expresses the above-mentioned TCR variant

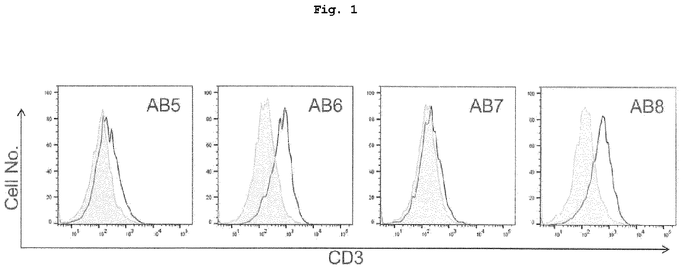

The T cells expressing each of the variants shown in Table 1 and obtained in [Example 2] were stained with an anti-CD3 antibody (APC/Cy7, UCHT1, BioLegend). Then, using LSR FortessaTMX-20 (BD Bioscience) flow cytometry, the expression of CD3 molecules on the cell membrane surface was detected by flow cytometry (Fig. 1). The expression of CD3 was also observed on the cell membrane surface of the cells obtained by AB5 and the cells obtained by AB7. In addition, the expression of CD3 was more strongly observed on the cell membrane surface of the cells obtained by AB6 and the cells obtained by AB8.

### [Example 3] Production of T cell derived from iPS cell expressing TCR variant

### 1. Preparation of iPS cell

As the iPS cell, iPS cells (Ff-I01s04 strain: derived from healthy human peripheral blood mononuclear cells) donated by the Center for iPS Cell Research and Application (CiRA), Kyoto University were used. The iPS cells were cultured according to the protocol "Feeder-free culture of human iPS cells" distributed by CiRA.

### 2. Production of Lenti V vector containing nucleic acid encoding variant of TCR having constant region of αβ TCR and not having complementarity determining region (CDR)

The sequence encoding the neomycin resistance gene was removed from pLVSIN-CMV Neo (Clontech), and a lentiviral vector using pLVSIN-Ub in which the CMV promoter was replaced with a human ubiquitin promoter was prepared. The artificial oligo DNA encoding AB6 synthesized above was incorporated into the multicloning site of the pLVSIN-Ub lentiviral vector. A lentiviral vector was prepared using this plasmid and Lenti-X™ 293T cell line and Lenti-X™ Packaging Single Shots (VSV-G) of Clontech.

### 3. Introduction of modified T cell receptor gene into iPS cell

A modified T cell receptor gene was introduced into iPS cells by infecting the cells with the lentiviral vector incorporating AB6 which was prepared in [Example 1].

A modified TCR gene was introduced into iPS cells by infecting the iPS cell prepared in [Example 3] 1. with the prepared lentivirus vector. In the following, the iPS cell into which the modified TCR gene has been introduced is sometimes referred to as "tTCR-iPSC".

### 4. Differentiation of iPS cell into hematopoietic progenitor cell (HPC)

Differentiation of iPS cell into hematopoietic progenitor cell (HPC) was performed according to a known method (e.g., method described in Cell Reports 2(2012)1722-1735 and WO 2017/221975). Specifically, tTCR-iPSC obtained in [Example 3] 3. was seeded in ultra-low adhesion-treated 6 well plates at 3 x 10⁵ cells/well, and cultured in EB medium (StemPro34 added with 10 µg/ml human insulin, 5.5 µg/ml human transferrin, 5 ng/ml sodium selenate, 2 mM L-glutamine, 45 mM α-monothioglycerol, and 50 µg/ml Ascorbic acid 2-phosphate) supplemented with 10 ng/ml BMP4, 50 ng/ml bFGF, 15 ng/ml VEGF, 2 µM SB431542 under low oxygen conditions (5% O₂) for 5 days. Subsequently, 50 ng/ml SCF, 30 ng/ml TPO, 10 ng/ml Flt3L were added, and the cells were further cultured for 5 - 9 days to obtain a non-adherent cell population. During the culture period, the medium was changed every 2 or 3 days. The above-mentioned non-adherent cell population containing HPC was stained using the antibody set in Table 2. The above-mentioned stained cell population was subjected to sorting by FACSAria.

**[Table 2]**

| | |
|---|---|
| anti-CD34 antibody | Abcam PE/Cy7 |
| anti-CD43 antibody | BD APC |
| anti-CD45 antibody | BioLegend BV510 |
| anti-CD14 antibody | BioLegend APC/eFluor780 |
| anti-CD235a antibody | BD FITC |

### 5. Differentiation of HPC into T cell

The cell fraction obtained in [Example 3] 4. was differentiated into lymphocytic cells according to a known method (e.g., methods described in Journal of Leukocyte Biology 96(2016)1165-1175 and WO 2017/221975). Specifically, a hematopoietic progenitor cell population was seeded at 2000 cells/well in a 48-well-plate coated with Recombinant h-DLL4/Fc chimera (SinoBiological) and Retronectin (Takara Bio Inc.), and cultured under 5% CO₂, 37°C conditions. During the culture period, the medium was changed every 2 or 3 days. As the medium, αMEM medium added with 15% FBS, 2 mM L-glutamine, 100 U/ml penicillin, 100 ng/ml streptomycin, 55 µM 2-mercaptoethanol, 50 µg/ml Ascorbic acid 2-phosphate, 10 µg/ml human insulin, 5.5 µg/ml human transferrin, 5 ng/ml sodium selenate, 50 ng/ml SCF, 50 ng/ml IL-7, 50 ng/ml Flt3L, 100 ng/ml TPO, 15 µM SB203580, 30 ng/ml SDF-1α was used. The cells were passaged in a similarly-coated 48-well-plate on days 7 and 14 from the start of culture. All cells were collected on day 21 from the start of culture. The collected cells were stained using the antibody set in Table 3.

**[Table 3]**

| | |
|---|---|
| CD3 antibody | Biolegend APC/Cy7 |
| CD5 antibody | BIolegend BV510 |
| CD7 antibody | Biolegend APC |
| TCRαβ antibody | eBIoscience FITC |

### [Experimental Example 2] Evaluation of cell membrane surface molecule expression of T cell that expresses the above-mentioned TCR variant

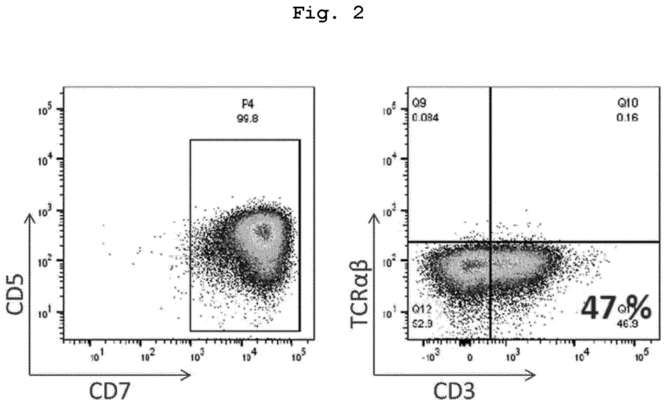

Expression of CD3, CD5, CD7, and αβTCR on the cell membrane surface by the cell obtained in [Example 3] 5. was measured with a flow cytometer (Fig. 2).

In the present specification, each term such as "comprising" or "comprise" is optionally replaced by "consisting of" or "consists of".

### [Industrial Applicability]

When expressed in cells, the variant of the present invention can suppress the alloreactivity of the cells, and thus can reduce the risk of graft-versus-host disease (GvHD) in allogeneic transplantation. That is, the introduction of the variant of the present invention can be one option for controlling the alloreactivity in T cell therapy.

This application is based on a patent application No. 2018-245253 filed in Japan (filing date: December 27, 2018), the contents of which are incorporated in full herein.

## Claims

1. A variant of a T-cell receptor comprising a combination of two polypeptides comprising a constant region of a T cell receptor chain selected from the group consisting of α chain, β chain, γ chain and δ chain, wherein the polypeptide does not comprise a complementarity determining region (CDR) of the T cell receptor chain, a complementarity determining region (CDR) of the α chain, and a complementarity determining region (CDR) of the β chain.

2. The variant according to claim 1, wherein one of the polypeptides comprises a constant region of the T cell receptor α chain or β chain, and the other comprises a constant region of the T cell receptor α chain or β chain.

3. The variant according to claim 1 or 2, wherein the two polypeptides are bound by one or more disulfide bonds.

4. A nucleic acid molecule encoding the variant according to any one of claims 1 to 3.

5. A vector comprising the nucleic acid molecule according to claim 4.

6. A method for producing a cell, comprising a step of introducing the vector according to claim 5.

7. A cell expressing the variant according to any one of claims 1 to 3.
